# EUROPEAN PATENT APPLICATION

(11) **EP 3 992 690 A1**
(43) Date of publication of application: **04.05.2022**
(21) Application number: 20851076.8
(22) Date of filing: 17.07.2020
(51) Int. Cl.: G02B 23/24, A61B 1/045

(54) **ENDOSCOPE SYSTEM, CONTROL DEVICE, AND CONTROL METHOD**

(30) Priority: 02.08.2019 JP 2019143033
(71) Applicant: Sony Group Corporation, Tokyo 108-0075 (JP)
(72) Inventor: KOBAYASHI, Motoaki, Tokyo 108-0075 (JP)
(74) Representative: D Young & Co LLP
(86) International application number: PCT/JP2020/027778
(87) International publication number: WO 2021/024754

(57) **Abstract**

The present disclosure relates to an endoscope system, a control device, and a control method capable of performing more satisfactory observation.

A cutout processing unit cuts out a part of an entire image obtained by imaging an entire field of view range of a rigid endoscope in accordance with a cutout region set around a position deviated from a center point of the entire image and generates an oblique image viewed in an oblique direction with respect to an optical axis of the rigid endoscope. An acquisition unit acquires an instruction command to move the oblique image in a predetermined rotation direction, and a region setting unit moves the cutout region around the center point of the entire image in a direction according to the instruction command along an inner periphery of a circle in which the cutout region is inscribed. The present technology is applicable to, for example, an endoscope system.

## Description

### TECHNICAL FIELD

The present disclosure relates to an endoscope system, a control device, and a control method and more particularly relates to an endoscope system, a control device, and a control method capable of performing more satisfactory observation.

### BACKGROUND ART

Endoscopic surgery has been conventionally performed as minimally invasive surgery. For example, in endoscopic surgery, it is possible to observe a lesion, an affected part to be treated, and the like by inserting an endoscope into a body from a small incision. At this time, a visually recognizable range using the endoscope is limited by an angle of view of the endoscope. Therefore, for example, in a case where the angle of view of the endoscope is about 60° to 90°, surgery is performed in a narrow field of view range. However, in order to safely perform surgery, it is desirable not only to see a video of a limited field of view range but also to observe the inside of the body in a wide range.

In view of this, Patent Document 1 proposes an ultra-wide-angle endoscope in which an ultra-wide-angle lens is attached to an endoscope to attempt to increase a field of view range without an operation of a scopist.

### CITATION LIST

### PATENT DOCUMENT

Patent Document 1: Japanese Patent No. 3070022

### SUMMARY OF THE INVENTION

### PROBLEMS TO BE SOLVED BY THE INVENTION

By the way, conventionally, a scopist who operates an endoscope changes a position and an angle of the endoscope to adjust a field of view direction so that the inside of a body can be observed in a wide range, thereby improving safety of surgery. However, in many cases, satisfactory observation depends on skills of the scopist, such as devising operation and proficiency through experience.

Further, in the configuration in which the ultra-wide-angle lens is attached to the endoscope as in Patent Document 1 described above, the field of view is distorted as a distance from a center of the lens increases. Therefore, generally, an image in which a center point of display is designated from a wide field of view and a certain range around the center point is subjected to distortion correction processing is displayed. However, in this case, the scopist needs to perform an operation of designating the center point of display by using a pointing device. Thus, the scopist simultaneously holds the endoscope with one hand and operates the pointing device. This requires a higher skill to perform satisfactory observation. Alternatively, in a case where an operator other than the scopist operates the pointing device, the number of surgical personnel increases, and, in addition, high cooperation is required between the scopist and the operator in order to perform satisfactory observation.

The present disclosure has been made in view of such a situation, and an object thereof is to perform more satisfactory observation.

### SOLUTIONS TO PROBLEMS

An endoscope system and a control device according to one aspect of the present disclosure include: a cutout processing unit configured to cut out a part of an entire image obtained by imaging an entire field of view range of a rigid endoscope in accordance with a cutout region set around a position deviated from a center point of the entire image and generate an oblique image viewed in an oblique direction with respect to an optical axis of the rigid endoscope; an acquisition unit configured to acquire an instruction command to move the oblique image in a predetermined rotation direction; and a region setting unit configured to move the cutout region around the center point of the entire image in a direction according to the instruction command along an inner periphery of a circle in which the cutout region is inscribed.

A control method according to one aspect of the present disclosure includes causing a control device of an endoscope system to cut out a part of an entire image obtained by imaging an entire field of view range of a rigid endoscope in accordance with a cutout region set around a position deviated from a center point of the entire image and generate an oblique image viewed in an oblique direction with respect to an optical axis of the rigid endoscope, to acquire an instruction command to move the oblique image in a predetermined rotation direction, and to move the cutout region around the center point of the entire image in a direction according to the instruction command along an inner periphery of a circle in which the cutout region is inscribed.

In one aspect of the present disclosure, a part of an entire image obtained by imaging an entire field of view range of a rigid endoscope is cut out in accordance with a cutout region set around a position deviated from a center point of the entire image and an oblique image viewed in an oblique direction with respect to an optical axis of the rigid endoscope is generated, an instruction command to move the oblique image in a predetermined rotation direction is acquired, and the cutout region is moved around the center point of the entire image in a direction according to the instruction command along an inner periphery of a circle in which the cutout region is inscribed.

### BRIEF DESCRIPTION OF DRAWINGS

Fig. 1 is a block diagram illustrating a configuration example of an embodiment of an endoscope system to which the present technology is applied.
Fig. 2 is an explanatory diagram of a relationship between an entire image and a forward image.
Fig. 3 is an explanatory diagram of a relationship between an entire image and an oblique image.
Fig. 4 is an explanatory diagram of display of an oblique image when a rotation operation is performed.
Fig. 5 illustrates an example of a display screen.
Fig. 6 is a block diagram illustrating a configuration example of a control device.
Fig. 7 is a flowchart showing an example of control processing.
Fig. 8 illustrates a second configuration example of an endoscope.
Fig. 9 illustrates a first variation of a display screen.
Fig. 10 illustrates a second variation of a display screen.
Fig. 11 illustrates a third variation of a display screen.
Fig. 12 illustrates a fourth variation of a display screen.
Fig. 13 illustrates a fifth variation of a display screen.
Fig. 14 is an explanatory diagram of a display method of stopping update of display content of an oblique image during a rotation operation.
Fig. 15 is an explanatory diagram of a display method of moving an oblique image so that a direction thereof rotates in response to a rotation operation.
Fig. 16 is an explanatory diagram of a display method of vertically inverting display content of an oblique image.
Fig. 17 is a block diagram illustrating a configuration example of an embodiment of a computer to which the present technology is applied.

### MODE FOR CARRYING OUT THE INVENTION

Hereinafter, specific embodiments to which the present technology is applied will be described in detail with reference to the drawings.

### <Configuration example of endoscope system>

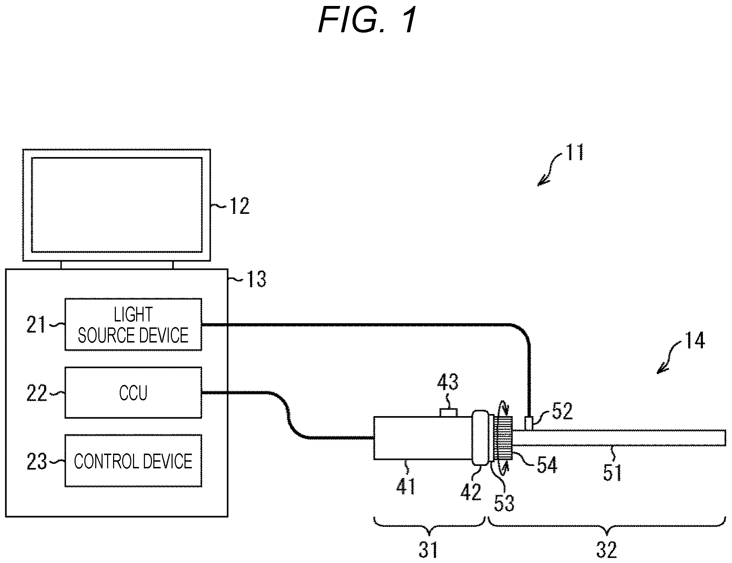

Fig. 1 is a block diagram illustrating a configuration example of an embodiment of an endoscope system to which the present technology is applied.

In Fig. 1, an endoscope system 11 is formed by mounting various devices for endoscopic surgery on a cart 13 on which a display device 12 is placed and can perform, for example, image processing on an image acquired by an endoscope 14 for use in endoscopic surgery.

The display device 12 displays an image acquired by the endoscope 14, an image obtained by performing image processing on the image, and the like.

In the example of Fig. 1, a light source device 21, a camera control unit (CCU) 22, and a control device 23 are mounted on the cart 13.

The light source device 21 includes, for example, an LED, a xenon lamp, a halogen lamp, a laser light source, or a light source corresponding to a combination thereof and supplies irradiation light to be emitted toward an observation target to the endoscope 14 through a light guide.

The CCU 22 controls image capturing by an imaging element included in a camera head 31, thereby supplying an image of the observation target captured by the imaging element.

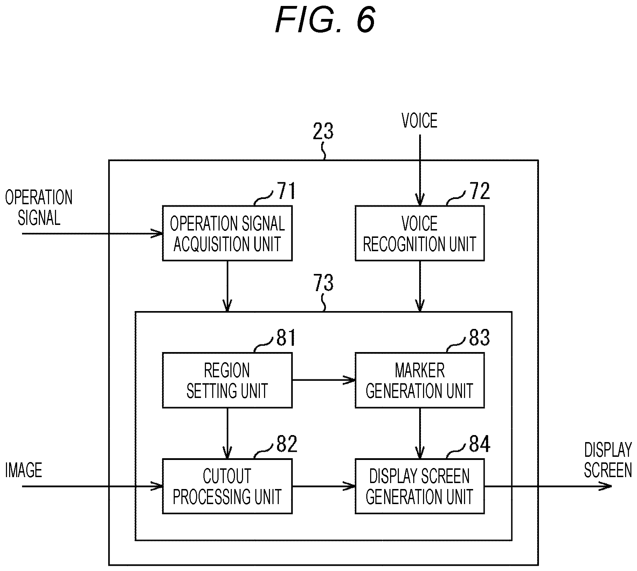

The control device 23 controls image processing on an image acquired by the endoscope 14. Note that a detailed configuration of the control device 23 will be described later with reference to Fig. 6.

The endoscope 14 is configured such that the camera head 31 and a rigid endoscope 32 are connectable and images an operative field at a high angle of view through the rigid endoscope 32.

The camera head 31 includes a camera head main body 41 including an imaging element for imaging an operative field, an endoscope coupler 42 used for connection to the rigid endoscope 32, and an operation switch 43 for inputting various operations.

The rigid endoscope 32 includes a lens barrel main body 51, a light source connection portion 52 to be connected to the light source device 21, a coupler connection portion 53 to be connected to the endoscope coupler 42 of the camera head 31, and a rotation operation portion 54 for inputting a rotation operation.

For example, the rotation operation portion 54 is provided in the vicinity of the endoscope coupler 42 to which the rigid endoscope 32 is attached and is configured such that a ring-shaped portion on an outer peripheral side is freely rotatable by a mechanism that rotates relative to the lens barrel main body 51. Further, the rotation operation portion 54 includes a rotary encoder that detects a rotation amount and a rotation speed of the ring-shaped portion.

The endoscope system 11 is configured as described above, and a surgeon, a scopist, and the like can, for example, perform a rotation operation on the rotation operation portion 54 while holding the endoscope 14. This makes it possible to stably hold the endoscope 14. Further, for example, in a conventional obliquely-viewing rotation method of rotating the rigid endoscope 32 itself, an attachment portion of the light guide arranged in the rigid endoscope is rotated together. Thus, it is necessary to become used to operation so as to prevent entanglement or the like of the light guide. Meanwhile, the rotation operation portion 54 is adopted in the endoscope system 11, and thus it is possible to perform a rotation operation on the rotation operation portion 54 while fixing a direction of the rigid endoscope 32. This does not require to become used to operation, unlike the conventional technology, and it is possible to make a learning curve extremely short.

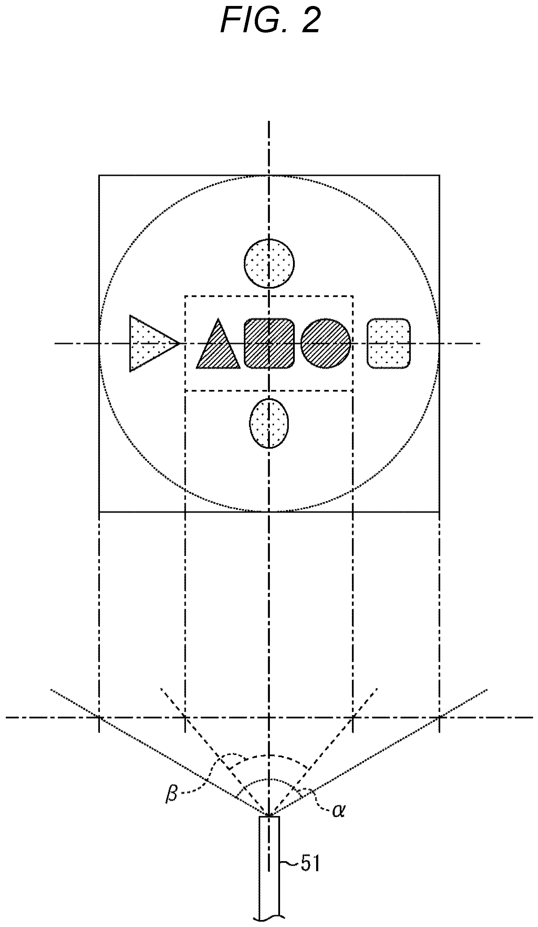

An entire image captured by the endoscope 14 and a forward image and an oblique image cut out from the entire image will be described with reference to Figs. 2 to 4.

In Fig. 2, a square region indicated by a solid line represents an imaging area of the imaging element of the endoscope 14. Further, a circular region indicated by a dotted line inscribed in the imaging area represents an entire field of view range imaged through the rigid endoscope 32, and an image of the field of view range is the entire image. That is, the entire image is a substantially circular image showing an effective area (area where no vignetting exists) of an image captured through the rigid endoscope 32, and the center of the effective area is a center point of the entire image.

Further, a rectangular region indicated by a broken line at the center of the entire image represents a cutout region showing a range of a forward image cut out from the entire image. That is, the forward image is generated by being cut out from the entire image in accordance with the cutout region set at the center of the entire image.

Further, a lower part of Fig. 2 illustrates a distal end portion of the lens barrel main body 51 of the rigid endoscope 32, and the rigid endoscope 32 has a geometrically wide angle of view. For example, an angle of view α of the entire image corresponding to the field of view (FOV) of the rigid endoscope 32 is approximately 140° or approximately 120°, and an angle of view β of the forward image cut out from the entire image corresponds to a range of approximately 80°.

Herein, the imaging element of the endoscope 14 is preferably equal to or more than 4096 × 2304 dots, which is so-called 4K resolution. That is, in order to provide a video having a sufficient resolution in surgery when a forward image or oblique image is cut out from the entire image, an imaging element of a conventional high definition (HD) resolution reduces the resolution when an image is cut out and displayed. Therefore, there is a concern that the imaging element of the conventional HD resolution cannot obtain a forward image or oblique image having a sufficient resolution to be used for endoscopic surgery. Thus, the imaging element of 4K resolution is adopted in the endoscope 14.

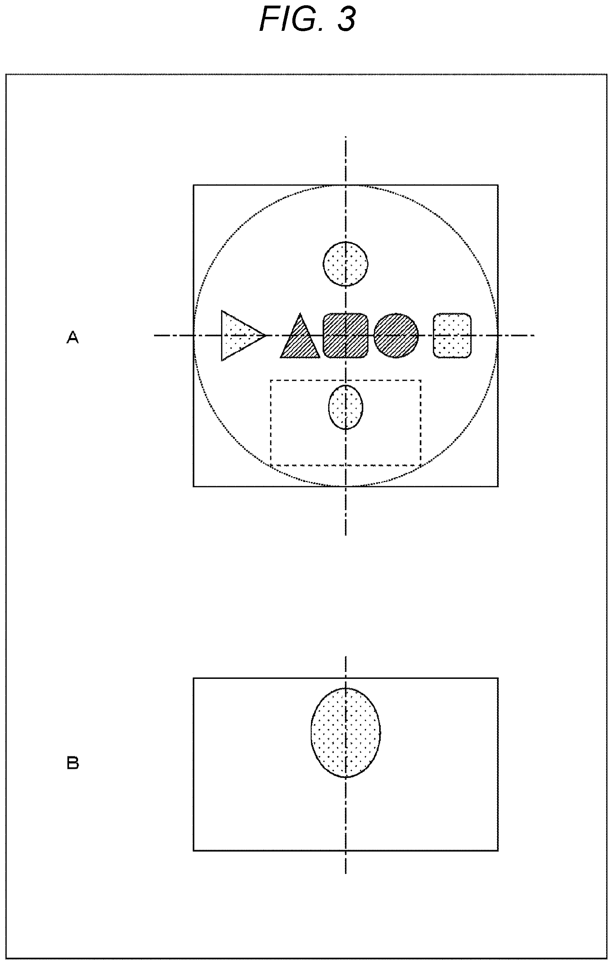

Further, a rectangular region indicated by a broken line in the entire image illustrated in A of Fig. 3 represents a cutout region showing a range of an oblique image cut out from the entire image. The cutout region of the oblique image has the same size as the cutout region of the forward image illustrated in Fig. 2 and is set at a position below the center of the entire image and in contact with an inner periphery of the entire image as illustrated in A of Fig. 3.

Further, B of Fig. 3 illustrates the oblique image generated by being cut out from the entire image in accordance with the cutout region of the oblique image. For example, the angle of view of the oblique image, as well as that of the forward image illustrated in Fig. 2, corresponds to a range of approximately 80°. Further, the oblique image cut out from the entire image in the cutout region set at the position in contact with the lower inner periphery of the entire image as illustrated in A of Fig. 3 corresponds to an oblique angle of approximately 30° from an optical axis in a downward direction. Note that, for example, the cutout region is only required to be set around a position deviated from the center point of the entire image and may be set at a position not in contact with the inner periphery of the entire image so as to correspond to an oblique angle of 30° or less.

Note that the value of the FOV described above is not limited to the value described herein and can be set to an appropriate value so as to satisfactorily perform observation by using the entire image or the oblique image. For example, the angle of view α of the entire image may correspond to a range of approximately 120°, and the angle of view β of the forward image or the oblique image may correspond to a range of approximately 80°. Herein, as illustrated in Fig. 3, the oblique image cut out from the entire image corresponds to an image captured by an oblique-viewing endoscope oriented in a straight downward direction in a confronting posture.

Further, in the endoscope system 11, it is possible to move the cutout region of the oblique image along the inner periphery of the entire image by performing a rotation operation on the rotation operation portion 54 of Fig. 1.

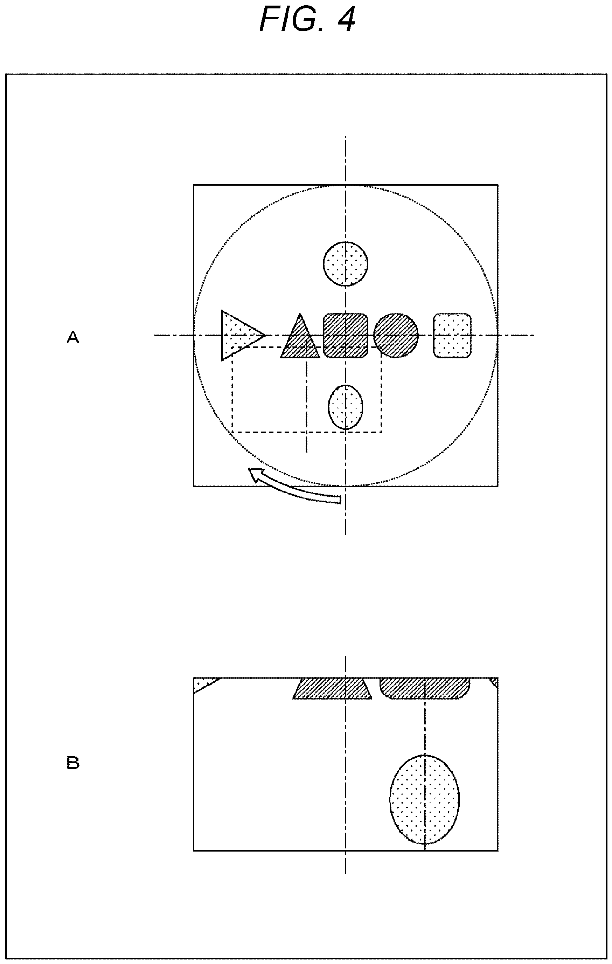

Fig. 4 illustrates a display example of the oblique image when the cutout region is moved along the inner periphery of the entire image.

For example, in a case where a rotation operation in a right rotation direction (clockwise direction) is performed on the rotation operation portion 54 while the oblique image in the straight downward direction is being displayed as illustrated in Fig. 3, the cutout region indicated by the broken-line rectangle moves around a forward central axis along the inner periphery of the entire image in the right rotation direction as illustrated in A of Fig. 4.

At this time, as illustrated in Fig. 4, the cutout region moves such that a moving direction of the cutout region rotates around the forward central axis while a direction of the cutout region does not rotate, for example, the cutout region moves while a vertical axis of the cutout region and a vertical axis of the entire image are being kept parallel and an outer shape of the cutout region is being in contact with the inner periphery of the entire image. As a result, as illustrated in B of Fig. 4, the oblique image cut out from the entire image in accordance with the cutout region is displayed. Note that, in a case where the cutout region is set at a position not in contact with the inner periphery of the entire image, the oblique image may be moved around the center point of the entire image along a circle in which the cutout region is inscribed.

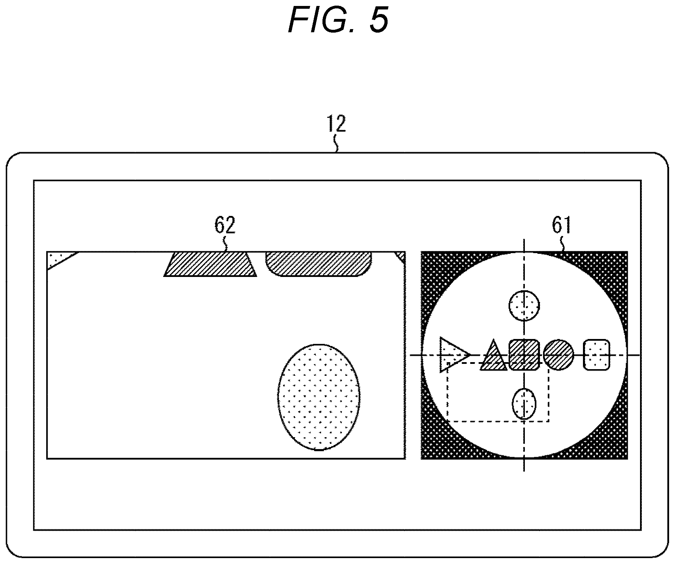

Fig. 5 illustrates an example of a display screen displayed on the display device 12.

On the display screen of Fig. 5, an entire image 61 showing the entire field of view range of the rigid endoscope 32 is arranged on a right side, and an oblique image 62 obtained by cutting out a part of the entire field of view range of the rigid endoscope 32 is arranged on a left side. Further, as indicated by a broken-line rectangle, a region marker indicating a cutout region of the oblique image 62 cut out from the entire field of view range of the rigid endoscope 32 is superimposed and displayed on the entire image 61 on the display screen.

In the endoscope system 11, such a display screen is displayed on the display device 12, and thus, for example, the surgeon, the scopist, and the like can simultaneously grasp the oblique image 62 showing an affected part to be closely observed and a state around the affected part from the entire image 61. Further, the region marker indicating the cutout region of the oblique image 62 is superimposed and displayed on the entire image 61, and thus the surgeon, the scopist, and the like can easily grasp a range and direction of the affected part to be closely observed with respect to the entire field of view range of the rigid endoscope 32.

For example, conventionally, in a case where forceps are inserted from a square area hatched with dots on a right side of the entire image 61, in a general oblique-viewing endoscope, the forceps appear in an image from the diagonal upper right after the forceps are inserted to some depth, and this may surprise the surgeon, the scopist, and the like.

Meanwhile, in the endoscope system 11, the surgeon, the scopist, and the like can confirm the forceps in the entire image 61 from when the forceps are started to be inserted. Further, in the endoscope system 11, the surgeon, the scopist, and the like can grasp that the oblique image 62 is located in a lower left direction of the entire image 61 from the region marker and can therefore recognize in advance that the forceps appear while being inserted from the upper right of the oblique image 62. Therefore, the surgeon, the scopist, and the like can accurately grasp the above situation and perform surgery.

As described above, even in a case where a certain direction is observed by using the oblique image 62, the endoscope system 11 simultaneously enables observation of the outside of the field of view of the oblique image 62 by using the entire image 61, that is, enables observation without generating a blind spot. Therefore, the surgeon, the scopist, and the like can perform surgery more safely by using the endoscope system 11.

### <Configuration example of control device and processing example of control processing>

Fig. 6 is a block diagram illustrating a configuration example of the control device 23.

As illustrated in Fig. 6, the control device 23 includes an operation signal acquisition unit 71, a voice recognition unit 72, and an image processing unit 73. Further, the image processing unit 73 includes a region setting unit 81, a cutout processing unit 82, a marker generation unit 83, and a display screen generation unit 84.

The operation signal acquisition unit 71 acquires an operation signal output when an operation is performed on the operation switch 43 and the rotation operation portion 54 of Fig. 1 and supplies an instruction command given as an instruction by the operation signal to the image processing unit 73. For example, the operation signal acquisition unit 71 acquires an operation signal according to a rotation operation on the rotation operation portion 54 and supplies, to the image processing unit 73, an instruction command to move the oblique image along the inner periphery of the entire image in the right rotation direction or a left rotation direction.

The voice recognition unit 72 receives supply of a voice uttered by the scopist via a microphone (not illustrated), performs voice recognition processing for recognizing the voice, and recognizes content of the utterance uttered by the scopist. Then, in a case where the voice recognition unit 33 recognizes an instruction command by voice on the basis of the content of the utterance from the scopist, the voice recognition unit 33 supplies the instruction command to the image processing unit 34. For example, based on a voice "Rotate the oblique image in the clockwise direction." uttered by the scopist, the voice recognition unit 72 can supply, to the image processing unit 73, an instruction command to move the oblique image along the inner periphery of the entire image in the clockwise direction. Thereafter, for example, based on a voice "Stop." uttered by the scopist when the image comes to a desired position to be observed, the voice recognition unit 72 can supply, to the image processing unit 73, an instruction command to stop the movement of the oblique image along the inner periphery of the entire image.

The image processing unit 73 performs image processing on an image captured by the endoscope 14 in accordance with an instruction command supplied from the operation signal acquisition unit 71 or the voice recognition unit 72 and causes the display device 12 to display the display screen as illustrated in Fig. 5.

The region setting unit 81 sets a cutout region that is cut out as a part of the entire image obtained by imaging the entire field of view range of the rigid endoscope 32. Then, the region setting unit 81 moves the cutout region around the center point of the entire image along the inner periphery of the entire image in a direction according to the instruction command.

The cutout processing unit 82 cuts out a part of the entire image supplied from the endoscope 14 in accordance with the cutout region set by the region setting unit 81, thereby generating an oblique image or forward image. For example, in a case where the cutout region is set around a position deviated from the center point of the entire image in accordance with the setting of the cutout region performed by the region setting unit 81, the cutout processing unit 82 generates an oblique image viewed in an oblique direction with respect to the optical axis of the rigid endoscope 32. Further, in a case where the setting of the cutout region performed by the region setting unit 81 is switched and the cutout region is set around a position at which a center point of the cutout region matches the center point of the entire image, the cutout processing unit 82 generates a forward image viewed in the same direction as the optical axis of the rigid endoscope 32.

The marker generation unit 83 generates a region marker indicating the cutout region set by the region setting unit 81, supplies the region marker to the display screen generation unit 84, and superimposes the region marker on the entire image.

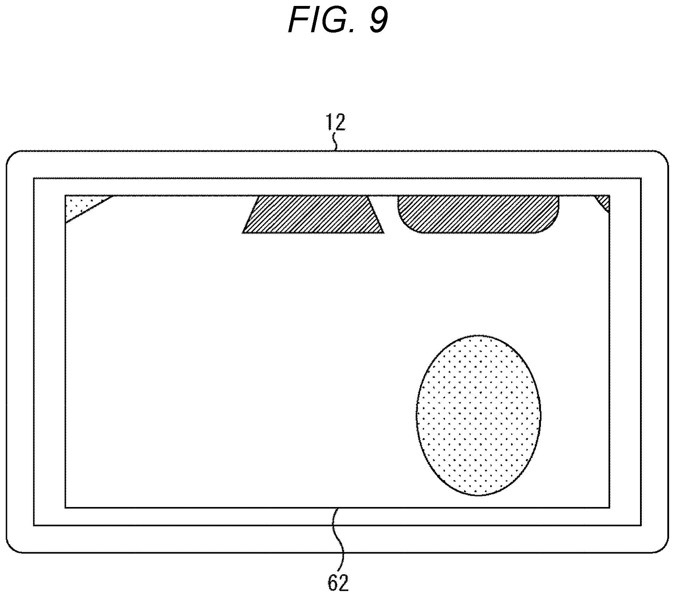

The display screen generation unit 84 generates, for example, the display screen displaying the entire image and the oblique image as illustrated in Fig. 5. Further, the display screen generation unit 84 generates various variations of the display screen as described later with reference to Figs. 9 to 13.

Herein, voice operation using the voice recognition unit 33 will be described.

For example, in a case where the scopist utters "Move the cutout region downward.", the setting of the cutout region is instantaneously changed to display an oblique image obtained by cutting out the position in the straight downward direction as illustrated in Fig. 3 described above even if the forward image is displayed at the time of the utterance or even if the oblique image shows any direction at the time of the utterance. That is, content of the utterance "Move the cutout region downward." is voice-recognized as an instruction command to move the cutout region to a position corresponding to 6 o'clock in the clockwise direction. Similarly, content of an utterance "Move the cutout region upward." is voice-recognized as an instruction command to move the cutout region to a position corresponding to 12 o'clock in the clockwise direction, content of an utterance "Move the cutout region leftward." is voice-recognized as an instruction command to move the cutout region to a position corresponding to 9 o'clock in the clockwise direction, and content of an utterance "Move the cutout region rightward." is voice-recognized as an instruction command to move the cutout region to a position corresponding to 3 o'clock in the clockwise direction.

By using such an instruction command, for example, it is possible to selectively use a case where the scopist utters "Rotate the cutout region in the clockwise direction." and desires to perform observation while gradually changing the field of view from a current field of view range toward a target position and a case where the scopist desires to instantaneously change the field of view from the current field of view range to the target position. This allows the scopist to easily obtain a more optimal field of view.

Further, for example, in a case where the scopist utters "Change the image to a forward image", it is possible to change the cutout region onto a forward axis and switch the field of view to a field of view equivalent to that obtained when a forward-viewing endoscope is used. Then, when obliquely-viewing rotation is started again, it is possible to change the image to an oblique image only by the scopist uttering "Change the image to an oblique image".

Herein, in the conventional endoscope, the rigid endoscope needs to be replaced in a case where it is desired to use the forward-viewing endoscope, and both the forward-viewing endoscope and the oblique-viewing endoscope need to be prepared and replaced as necessary. Therefore, conventionally, time loss occurs due to an effort to secure a place for arranging both the forward-viewing endoscope and the oblique-viewing endoscope and replace the forward-viewing endoscope and the oblique-viewing endoscope.

Meanwhile, the endoscope system 11 can provide both functions of the forward-viewing endoscope and the oblique-viewing endoscope by using one rigid endoscope 32 and can switch the forward image and the oblique image as necessary. Therefore, it is possible to perform surgery while avoiding the occurrence of the time loss due to the effort to replace the forward-viewing endoscope and the oblique-viewing endoscope, without needing to secure a place for arranging both the forward-viewing endoscope and the oblique-viewing endoscope.

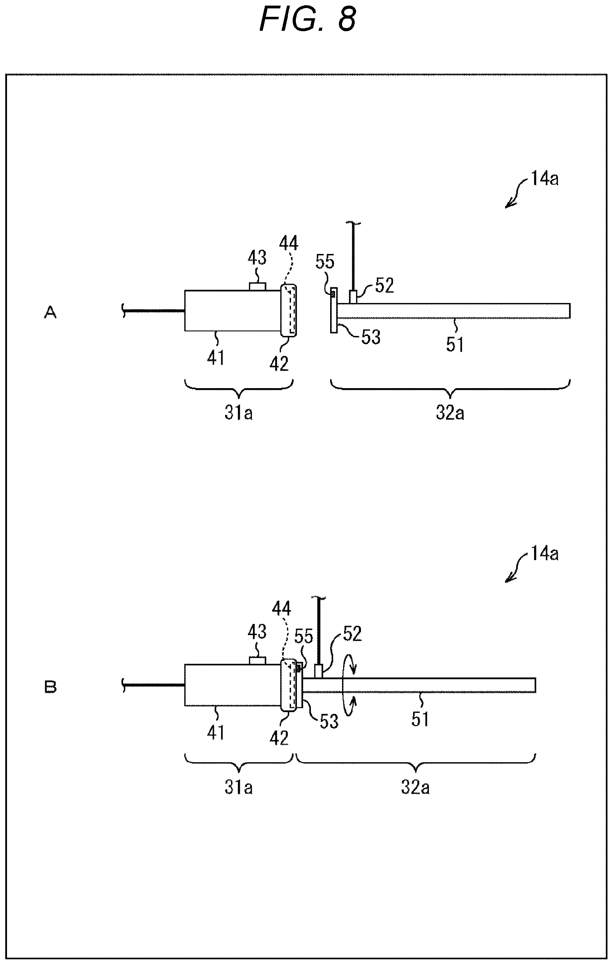

Further, this voice operation and an operation method using the rotation operation portion 54 of Fig. 1 or a combination of a magnetic encoder 44 and a magnet 55 of Fig. 8 may be simultaneously used. In that case, for example, it is also possible to provide a usage in which the surgeon uses voice operation as necessary while the scopist directly performs operation by hand.

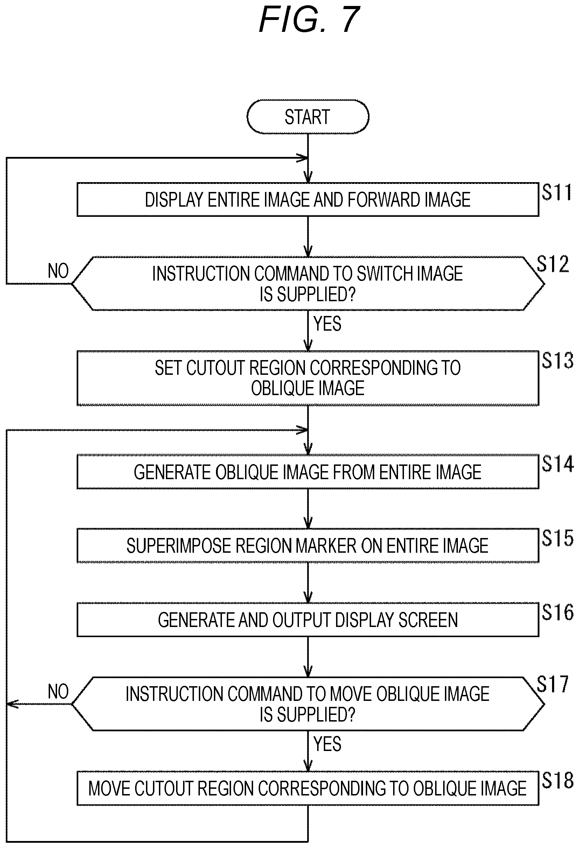

An example of control processing executed by the control device 23 will be described with reference to the flowchart of Fig. 7.

In step S11, the control device 23 displays, on the display device 12, an entire image captured by the endoscope 14 showing the entire field of view range of the rigid endoscope 32 and a forward image obtained by cutting out the center of the entire image. That is, in the control device 23, the region setting unit 81 sets a cutout region at the center of the entire image (see Fig. 2) supplied from the endoscope 14, and the cutout processing unit 82 cuts out a forward image from the entire image in accordance with the cutout region, thereby generating the forward image. Then, the display screen generation unit 84 generates, for example, a display screen on which the entire image and the forward image are arranged side by side and outputs the display screen to the display device 12.

In step S12, the operation signal acquisition unit 71 determines, for example, whether or not an instruction command to switch the forward image to an oblique image has been supplied by operating the operation switch 43. In a case where the operation signal acquisition unit 71 determines in step S12 that the instruction command to switch the forward image to an oblique image has not been supplied, the processing returns to step S11, and the processing enters a standby state.

Meanwhile, in a case where the operation signal acquisition unit 71 determines in step S12 that the instruction command to switch the forward image to an oblique image has been supplied, the processing proceeds to step S13.

In step S13, the region setting unit 81 sets a cutout region corresponding to the oblique image at, for example, a position in the straight downward direction as illustrated in Fig. 3 as an initial position of the cutout region. Then, the region setting unit 81 notifies the cutout processing unit 82 and the marker generation unit 83 of the set cutout region.

In step S14, the cutout processing unit 82 cuts out an oblique image from the entire image in accordance with the cutout region set by the region setting unit 81 in step S13, thereby generating the oblique image.

In step S15, the marker generation unit 83 generates a region marker indicating the cutout region set by the region setting unit 81 in step S13, supplies the region marker to the display screen generation unit 84, and superimposes the region marker on the entire image.

In step S16, the display screen generation unit 84 generates a display screen on which the oblique image generated in step S14 and the entire image on which the region marker is superimposed are arranged side by side and outputs the display screen to the display device 12. Therefore, the display of the display device 12 is switched from the forward image to the oblique image.

In step S17, the operation signal acquisition unit 71 determines, for example, whether or not an instruction command to move the oblique image along the inner periphery of the entire image has been supplied by performing a rotation operation on the rotation operation portion 54.

In a case where the operation signal acquisition unit 71 determines in step S17 that the instruction command to move the oblique image along the inner periphery of the entire image has not been supplied, the processing returns to step S14, and similar processing is repeatedly performed in accordance with the cutout region set at the current time.

Meanwhile, in a case where the operation signal acquisition unit 71 determines in step S17 that the instruction command to move the oblique image along the inner periphery of the entire image has been supplied, the processing proceeds to step S18.

In step S18, the region setting unit 81 moves the cutout region corresponding to the oblique image along the inner periphery of the entire image in accordance with a rotation amount and a rotation speed of the rotation operation on the rotation operation portion 54, thereby newly setting a cutout region. Thereafter, the processing returns to step S14, and the similar processing is repeatedly performed in accordance with the newly set cutout region, and the oblique image moved along the inner periphery of the entire image in response to the rotation operation on the rotation operation portion 54 is displayed.

As described above, the control device 23 can switch display between the forward image and the oblique image in response to the operation on the operation switch 43. Then, when displaying the oblique image, the control device 23 can display the entire image on which the region marker corresponding to the cutout region in which the oblique image is cut out is superimposed. Then, the control device 23 can move the oblique image along the inner periphery of the entire image in response to the rotation operation on the rotation operation portion 54.

Note that the control device 23 can perform distortion correction on the oblique image generated by the cutout processing unit 82 in accordance with a position of the cutout region with respect to the entire image. For example, the oblique image moves around the center point of the entire image along the inner periphery of the entire image, and non-uniform distortion may occur in the entire image due to a manufacturing error or the like of a wide-angle lens. Therefore, it is possible to obtain a more satisfactory oblique image by calibrating the wide-angle lens before shipping of the control device 23 and performing distortion correction on the oblique image in accordance with a position of the cutout region on the basis of the calibration information.

### <Second configuration example of endoscope>

A second configuration example of the endoscope 14 will be described with reference to Fig. 8. Note that the same configurations of an endoscope 14a of the second configuration example illustrated in Fig. 8 as those of the endoscope 14 of the first configuration example illustrated in Fig. 1 will be denoted by the same reference signs, and detailed description thereof will be omitted.

As illustrated in Fig. 8, the endoscope 14a is configured such that a rigid endoscope 32a is attachable to a camera head 31a. A of Fig. 8 illustrates a state in which the rigid endoscope 32a is detached from the camera head 31a, and B of Fig. 8 illustrates a state in which the rigid endoscope 32a is attached to the camera head 31a.

The camera head 31a, as well as the camera head 31 of Fig. 1, includes the camera head main body 41, the endoscope coupler 42, and the operation switch 43 and further includes the magnetic encoder 44. The rigid endoscope 32a, as well as the rigid endoscope 32 of Fig. 1, includes the lens barrel main body 51, the light source connection portion 52, and the coupler connection portion 53 and further includes the magnet 55.

The magnetic encoder 44 is incorporated in the endoscope coupler 42 and is a magnetic detection means for detecting magnetism of the magnet 55. The magnet 55 is incorporated in the coupler connection portion 53. Then, when the rigid endoscope 32a is rotated with respect to the camera head 31a, the magnetic encoder 44 detects the magnetism of the magnet 55 to recognize a rotation direction and a rotation amount, and an operation signal indicating the rotation direction and the rotation amount is supplied to the operation signal acquisition unit 71. Therefore, the control device 23 can control movement of the oblique image along the inner periphery of the entire image in response to the rotation operation of rotating the rigid endoscope 32a with respect to the camera head 31a.

Note that the endoscope coupler 42 having a similar structure to that of a conventional endoscope coupler is adopted in the endoscope 14a, and therefore, for example, a conventional rigid endoscope in which the magnet 55 is not incorporated in the coupler connection portion 53 can be used by being attached to the endoscope coupler 42. In a case where the conventional rigid endoscope is connected to the endoscope coupler 42 as described above, the magnetic encoder 44 does not detect magnetism. Therefore, in this case, the control device 23 does not perform the processing of cutting out an oblique image from the entire image, and an image captured by the rigid endoscope is displayed on the display device 12 as in a conventional case.

In the endoscope system 11 including the endoscope 14a having such a configuration, when one of the endoscope 14a and the conventional rigid endoscope is attached, the control device 23 can automatically provide a function of the attached endoscope. That is, in a case where the endoscope 14a is attached, the endoscope system 11 can provide a function of moving the oblique image along the inner periphery of the entire image in accordance with a rotation operation of the rigid endoscope 32a with respect to the camera head 31a, whereas, in a case where the conventional rigid endoscope is attached, the endoscope system 11 can provide a function corresponding to the rigid endoscope. Therefore, the surgeon, the scopist, and the like can use the endoscope system 11 including the conventional rigid endoscope without discomfort. Therefore, a learning curve for acquiring an operation method of the endoscope system 11 is short, and a conventional rigid endoscope can also be used in the endoscope system 11. This contributes to health economy.

### <Variations of display screen>

Variations of the display screen displayed on the display device 12 will be described with reference to Figs. 9 to 13.

In a first variation of the display screen illustrated in Fig. 9, only the oblique image 62 obtained by cutting out a part of the entire field of view range of the rigid endoscope 32 is arranged on the entire screen. With this display screen, the surgeon, the scopist, and the like can perform surgery while concentrating on the oblique image 62 displayed in a large size and observing only the oblique image 62. This display screen is effective in such a situation. Note that, because the entire image 61 and the oblique image 62 are not simultaneously displayed on this display screen, peripheral observation using the entire image 61 cannot be simultaneously performed. However, for example, it is only required to instantaneously change the display screen to a display screen on which the entire image 61 and the oblique image 62 are displayed side by side by issuing a switching instruction by voice operation.

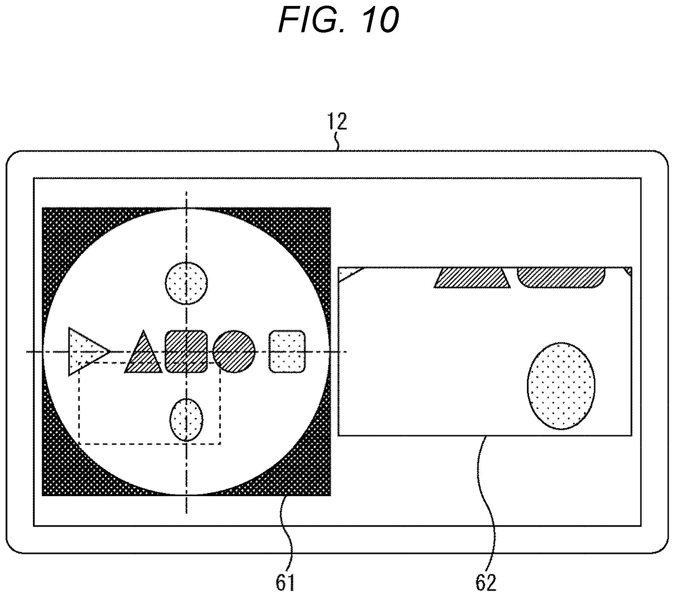

In a second variation of the display screen illustrated in Fig. 10, the entire image 61 is arranged on a left side, and the oblique image 62 is arranged on a right side. Further, a display size of the entire image 61 is adjusted to have a height corresponding to a height of the display device 12, and a display size of the oblique image 62 is adjusted to have a width corresponding to a remaining width of the display device 12 displaying the entire image 61.

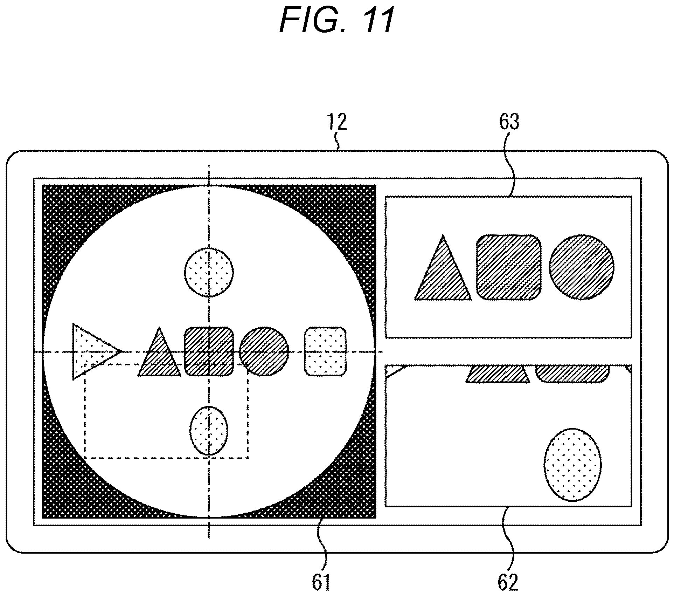

In a third variation of the display screen illustrated in Fig. 11, the entire image 61 is arranged on a left side, and, on a remaining right side, the oblique image 62 is arranged on a lower side and a forward image 63 is arranged on an upper side.

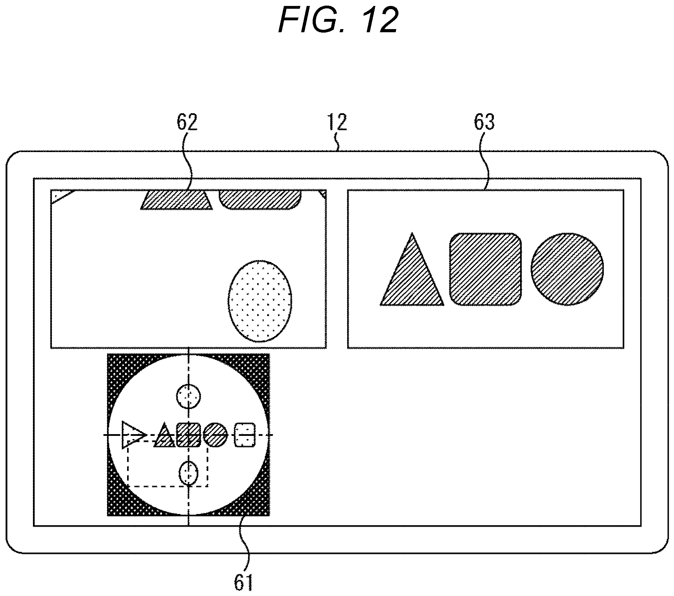

In a fourth variation of the display screen illustrated in Fig. 12, the oblique image 62 on an upper left side and the forward image 63 on the upper left side are arranged side by side, and the entire image 61 is arranged below the oblique image 62.

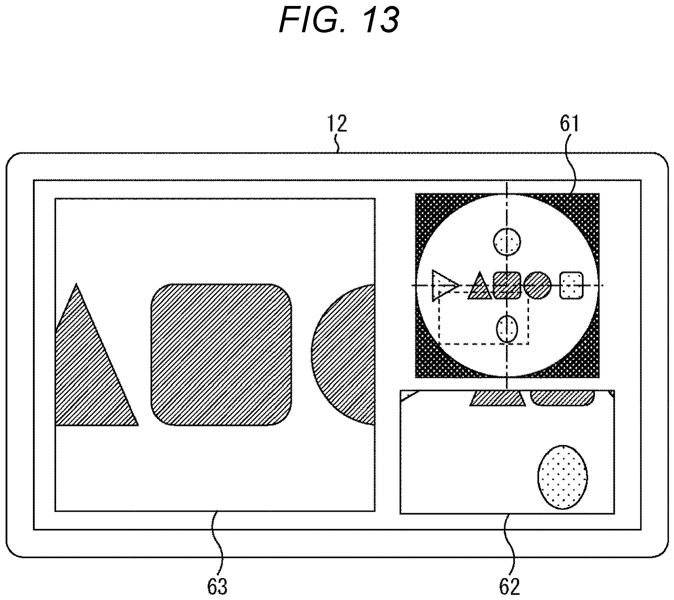

In a fifth variation of the display screen illustrated in Fig. 13, the forward image 63 cut into a square shape is arranged on a left side in accordance with the height of the display device 12, and, on a remaining right side, the entire image 61 is arranged on an upper side and the oblique image 62 is arranged on a lower side.

As described above, the endoscope system 11 can display various variations of the display screen illustrated in Figs. 9 to 13 on the display device 12. Therefore, for example, it is possible to flexibly change the variation of the display screen in accordance with the kind of surgery, an intention of the surgeon, or the like. This makes it possible to optimally use the endoscope system in many clinical departments, as compared with the conventional endoscope. For example, it is also possible to provide a value of equipping a general hospital to use the endoscope system 11 in common, instead of preparing the endoscope system for each clinical department.

### <Variations of method of displaying oblique image>

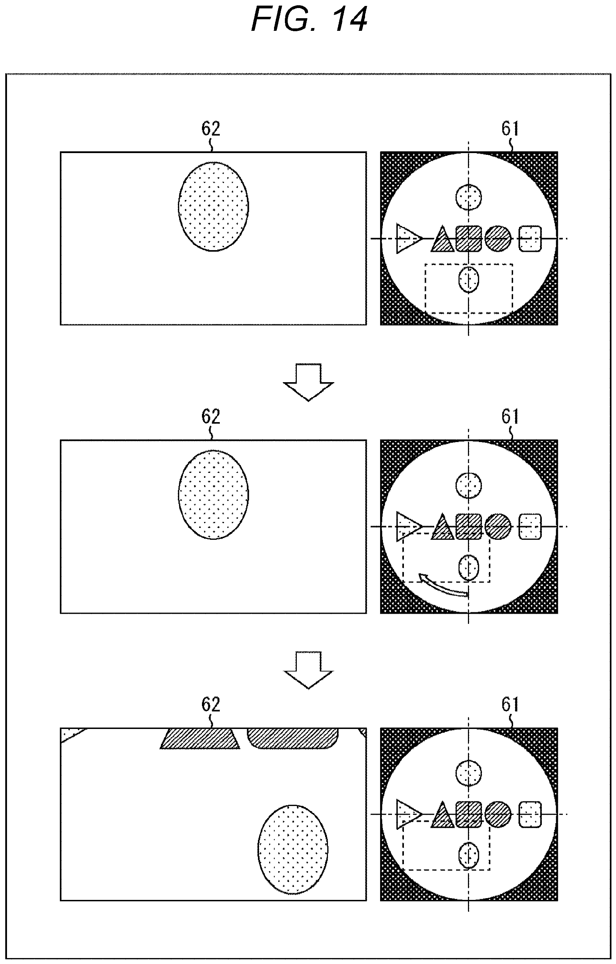

Variations of a method of displaying an oblique image will be described with reference to Figs. 14 to 16.

For example, the endoscope system 11 generally adopts a display method of updating, in real time, display of the oblique image 62 whose display content is a part cut out from the entire image 61 in accordance with the cutout region set to move along the inner periphery of the entire image in response to a rotation operation on the oblique image 62.

Meanwhile, in a display method described with reference to Fig. 14, the update of the display content of the oblique image 62 is stopped when a rotation operation on the oblique image 62 is started, and the display content immediately before the rotation operation is performed is held. Then, when the rotation operation on the oblique image 62 ends, the oblique image 62 is displayed to have display content switched in accordance with the cutout region set by moving along the inner periphery of the entire image in response to the rotation operation.

First, as illustrated in an upper part of Fig. 14, when no rotation operation is performed on the oblique image 62, the oblique image 62 displays display content corresponding to the cutout region superimposed and displayed on the entire image 61.

Then, as illustrated in a middle part of Fig. 14, while a rotation operation is being performed on the oblique image 62, a display position of the region marker moves along the inner periphery of the entire image in response to the rotation operation, whereas the update of the oblique image 62 is stopped to hold the display content immediately before the rotation operation is performed. That is, the region setting unit 81 sets the cutout region so that the cutout region moves along the inner periphery of the entire image in response to the rotation operation, and the marker generation unit 83 updates display of the region marker in accordance with the cutout region, whereas the cutout processing unit 82 does not cut out the oblique image 62. Therefore, the oblique image 62 holds the display content immediately before the rotation operation is performed on the oblique image 62, i.e., in this example, the same display content as that in the upper part of Fig. 14.

Thereafter, as illustrated in a lower part of Fig. 14, at a timing when the rotation operation on the oblique image 62 ends (e.g., after one second from the end of the rotation operation), the oblique image 62 displays display content corresponding to the cutout region superimposed and displayed on the entire image 61 at that time. That is, the region setting unit 81 sets the cutout region at a position at which the movement along the inner periphery of the entire image ends in response to the end of the rotation operation, and the cutout processing unit 82 cuts out display content according to the cutout region from the entire image 61, and thus the oblique image 62 is displayed.

According to such a display method, the scopist can move the cutout region to a desired region while recognizing display content to be displayed as the oblique image 62 at the end of the rotation operation on the basis of the region marker moving on the entire image 61. Further, the image processing of cutting out the oblique image in the cutout processing unit 82 is stopped while the rotation operation is being performed. This makes it possible to greatly reduce a processing load of the image processing unit 73. Therefore, for example, it is possible to achieve cost reduction of the image processing unit 73.

Note that a modification example of this display method is a display method of displaying a low-resolution oblique image 62 to intentionally reduce a resolution of the oblique image 62, instead of stopping the update of the display content of the oblique image 62, while a rotation operation is being performed on the oblique image 62 and displaying the oblique image 62 having an original resolution when the rotation operation on the oblique image 62 ends. In such a display method, it is possible to reduce the processing load of the image processing unit 73 and to easily recognize that the rotation operation is performed because the display content of the oblique image 62 changes.

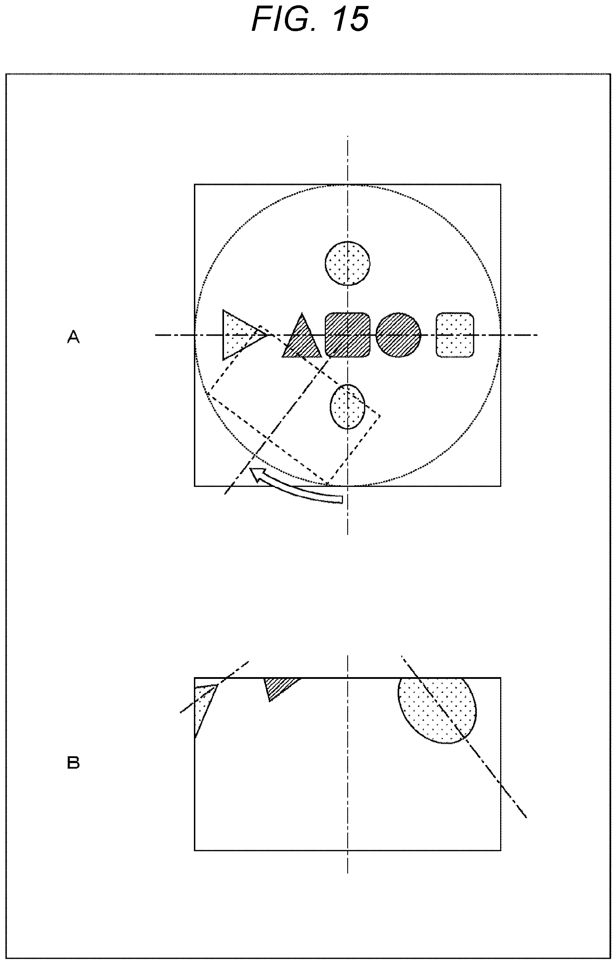

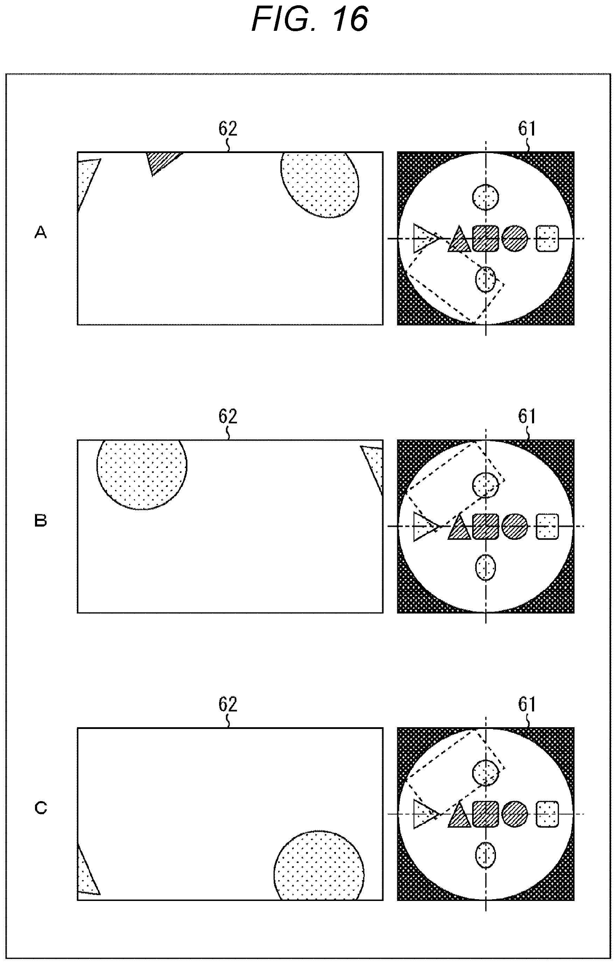

Next, in a display method described with reference to Figs. 15 and 16, for example, in a case where the cutout region of the oblique image is moved along the inner periphery of the entire image in response to a rotation operation on the rotation operation portion 54 of Fig. 1, the cutout region is set so that a direction of the oblique image rotates in accordance with the movement. That is, as illustrated in A of Fig. 15, the cutout region moves along the inner periphery of the entire image so as to rotate around the forward central axis serving as the center of rotation while both lower corners of the cutout region are being in constant contact with the inner periphery of the entire image. As a result, as illustrated in B of Fig. 15, the oblique image cut out from the entire image in accordance with the cutout region is displayed.

By the way, in a case where the direction of the oblique image rotates, as described with reference to Fig. 16, the oblique image 62 can be displayed so that the display content thereof is vertically inverted in accordance with a position of the cutout region.

For example, as illustrated in A of Fig. 16 and B of Fig. 16, the display content of the oblique image 62 cut out from the entire image 61 is generally displayed in the following vertical direction, regardless of the position of the cutout region: an outer peripheral side of the entire image 61 is on the bottom and a center side of the entire image 61 is on the top.

Meanwhile, in a case where the center of the cutout region is at a position below a horizontal line passing through the center of the entire image 61 as illustrated in A of Fig. 16, the oblique image 62 is displayed in the normal vertical direction. However, in a case where the center of the cutout region is at a position above the horizontal line passing through the center of the entire image 61 as illustrated in C of Fig. 16, the oblique image 62 is displayed in an inverted direction to the normal vertical direction. That is, the display content of the oblique image 62 can be displayed so as to be vertically inverted depending on whether the center of the cutout region is above or below the horizontal line passing through the center of the entire image 61.

According to such a display method, for example, in a case where the cutout region is set immediately above the center of the entire image 61, a vertical direction in the region marker superimposed on the entire image 61 matches the vertical direction of the display content of the oblique image 62. Therefore, a relationship between the region marker superimposed on the entire image 61 and the oblique image 62 can be more easily recognized.

Note that the endoscope system 11 is not limited to the configuration including a single display device 12 as illustrated in Fig. 1 and may adopt a configuration including a plurality of display devices 12. For example, in a configuration in which two display devices 12 are provided, one display device 12 has the display screen on which only the oblique image 62 is arranged as illustrated in Fig. 9, and the other display device 12 has the display screen on which the entire image 61 and the oblique image 62 are arranged side by side as illustrated in Fig. 5. Therefore, the surgeon can perform surgery while viewing the display screen on which only the oblique image 62 is arranged, and the scopist can safely operate the endoscope 14 while viewing the display screen on which the entire image 61 and the oblique image 62 are arranged side by side. This makes it possible to effectively use the endoscope system 11.

### <Configuration example of computer>

Next, the series of processing (control method) described above can be performed by hardware or software. In a case where the series of processing is executed by software, a program forming the software is installed in a general-purpose computer or the like.

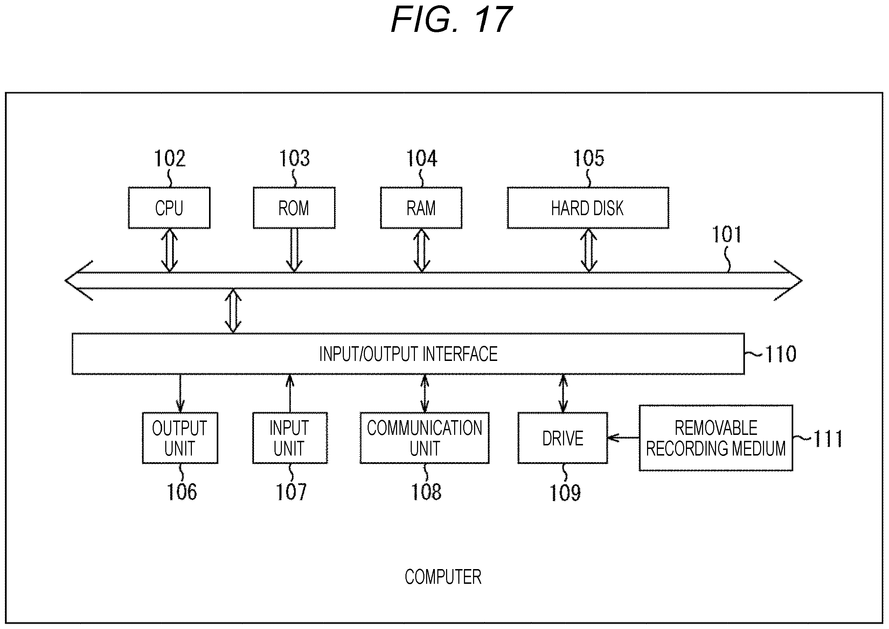

Fig. 17 is a block diagram illustrating a configuration example of an embodiment of a computer in which a program for executing the series of processing described above is installed.

The program can be recorded in advance on a hard disk 105 or ROM 103 serving as a recording medium included in the computer.

Alternatively, the program can be stored (recorded) in a removable recording medium 111 driven by a drive 109. Such the removable recording medium 111 can be provided as so-called packaged software. Herein, examples of the removable recording medium 111 encompass a flexible disk, a compact disc read only memory (CD-ROM), a magneto optical (MO) disk, a digital versatile disc (DVD), a magnetic disk, and a semiconductor memory.

Note that the program can be installed in the computer from the removable recording medium 111 described above or can be downloaded to the computer via a communication network or broadcast network and be installed in the built-in hard disk 105. That is, for example, the program can be wirelessly transferred from a download site to the computer via an artificial satellite for digital satellite broadcasting or can be transferred by wire to the computer via a network such as a local area network (LAN) or the Internet.

The computer includes a central processing unit (CPU) 102, and the CPU 102 is connected to an input/output interface 110 via a bus 101.

When a user inputs a command via the input/output interface 110 by, for example, operating an input unit 107, the CPU 102 executes a program stored in the read only memory (ROM) 103 in response to the command. Alternatively, the CPU 102 loads the program stored in the hard disk 105 into a random access memory (RAM) 104 and executes the program.

Therefore, the CPU 102 performs the processing according to the flowchart described above or the processing performed by the configuration of the block diagram described above. Then, for example, the CPU 102 outputs the processing result from an output unit 106 or transmits the processing result from a communication unit 108 via the input/output interface 110 and further, for example, records the processing result on the hard disk 105, as necessary.

Note that the input unit 107 includes a keyboard, a mouse, a microphone, and the like. Further, the output unit 106 includes a liquid crystal display (LCD), a speaker, and the like.

Herein, in the present specification, the processing performed by the computer according to the program is not necessarily performed in time series in the order shown in the flowchart. That is, the processing performed by the computer according to the program also includes processing executed in parallel or individually (e.g., parallel processing or processing by an object).

Further, the program may be processed by a single computer (processor) or may be processed in a distributed manner by a plurality of computers. Further, the program may be transferred to a remote computer and be executed therein.

Further, in the present specification, a system means a set of a plurality of components (devices, modules (parts), and the like), and it does not matter whether or not all the components are included in the same housing. Therefore, a plurality of devices included in separate housings and connected via a network and a single device including a plurality of modules in a single housing are both systems.

Further, for example, a configuration described as a single device (or processing unit) may be divided and configured as a plurality of devices (or processing units). On the contrary, in the above description, a configuration described as a plurality of devices (or processing units) may be integrally configured as a single device (or processing unit). Further, as a matter of course, a configuration other than the configurations described above may be added to the configuration of each device (or each processing unit). Furthermore, a part of a configuration of a certain device (or processing unit) may be included in a configuration of another device (or another processing unit) as long as a configuration or operation of the entire system is substantially the same.

Further, for example, the present technology can have a configuration of cloud computing in which a single function is shared and jointly processed by a plurality of devices via a network.

Further, for example, the program described above can be executed in an arbitrary device. In that case, the device is only required to have a necessary function (e.g., a functional block) to obtain necessary information.

Further, for example, each of the steps described in the above flowchart can be executed by a single device or can be executed by being shared by a plurality of devices. Furthermore, in a case where a single step includes a plurality of processes, the plurality of processes included in the single step can be executed by a single device or can be executed by being shared by a plurality of devices. In other words, the plurality of processes included in the single step can also be executed as processes in a plurality of steps. On the contrary, the processes described as the plurality of steps can also be integrally executed as a single step.

Note that, in the program executed by the computer, processes in steps describing the program may be executed in time series in the order described in this specification or may be executed in parallel or individually at a necessary timing such as when a call is made. That is, the processes in the respective steps may be executed in order different from the order described above as long as there is no contradiction. Further, the processes in the steps describing the program may be executed in parallel with processes of another program or may be executed in combination with processes of another program.

Note that a plurality of the present technologies described in the present specification can each be implemented alone independently as long as there is no contradiction. As a matter of course, a plurality of arbitrary present technologies can also be implemented in combination. For example, a part of or the entire present technology described in any embodiment can be implemented in combination with a part of or the entire present technology described in another embodiment. Further, a part of or the entire arbitrary present technology described above can also be implemented in combination with another technology not described above.

### <Combination examples of configurations>

Note that the present technology can also have the following configurations.
(1) An endoscope system including:
   a cutout processing unit configured to cut out a part of an entire image obtained by imaging an entire field of view range of a rigid endoscope in accordance with a cutout region set around a position deviated from a center point of the entire image and generate an oblique image viewed in an oblique direction with respect to an optical axis of the rigid endoscope;
   an acquisition unit configured to acquire an instruction command to move the oblique image in a predetermined rotation direction; and
   a region setting unit configured to move the cutout region around the center point of the entire image in a direction according to the instruction command along an inner periphery of a circle in which the cutout region is inscribed.
(2) The endoscope system according to (1), further including:
   a display screen generation unit configured to generate a display screen displaying at least one of the entire image or the oblique image; and
   a marker generation unit configured to generate a region marker indicating the cutout region in the entire image and superimpose and display the region marker on the entire image displayed on the display screen.
(3) The endoscope system according to (1) or (2), further including:
   an endoscope configured to image an operative field at a high angle of view through the rigid endoscope and supply the entire image to the cutout processing unit.
(4) The endoscope system according to (3), in which
   the endoscope is configured such that the rigid endoscope is connectable to a camera head including an imaging element that images the operative field.
(5) The endoscope system according to (4), in which
   the rigid endoscope includes a rotation operation portion for inputting a rotation operation to move the oblique image in the vicinity of a connection portion used for connection to the camera head, and
   the acquisition unit acquires the instruction command in response to the rotation operation on the rotation operation portion.
(6) The endoscope system according to (4), in which
   the camera head includes a coupler used for connection to the rigid endoscope,
   the rigid endoscope includes a connection portion to be connected to the coupler,
   a magnet is incorporated in one of the connection portion and the coupler, and a magnetic encoder is incorporated in the other of the connection portion and the coupler, and
   the acquisition unit acquires the instruction command in response to a rotation operation of rotating the rigid endoscope with respect to the camera head.
(7) The endoscope system according to any one of (1) to (6), in which
   the acquisition unit is a voice recognition unit that acquires the instruction command by recognizing content of an utterance from a voice.
(8) The endoscope system according to any of (1) to (7), in which
   the region setting unit switches and sets the cutout region around the position deviated from the center point of the entire image and a cutout region around a position at which a center point of the cutout region matches the center point of the entire image, and
   the cutout processing unit generates the oblique image on the basis of the cutout region set around the position deviated from the center point of the entire image and generates a forward image on the basis of the cutout region set around the position at which the center point of the cutout region matches the center point of the entire image.
(9) The endoscope system according to any of (1) to (8), in which
   an angle of view of the entire image is approximately 140° or approximately 120°, and a resolution of the entire image is equal to or more than 4096 × 2304 dots.
(10) The endoscope system according to any of (1) to (9), in which
   distortion correction is performed on the oblique image in accordance with a position of the cutout region with respect to the entire image.
(11) The endoscope system according to any of (1) to (10), in which
   the center point of the entire image is a center of an effective area of an image captured through the rigid endoscope.
(12) The endoscope system according to any of (1) to (11), in which
   the cutout region is set so that a direction of the oblique image rotates as the cutout region moves, and
   display content of the oblique image is displayed so as to be vertically inverted depending on a position of the cutout region.
(13) The endoscope system according to (12), in which
   the display content of the oblique image is vertically inverted depending on whether the position of the cutout region is above or below a horizontal line passing through the center point of the entire image.
(14) The endoscope system according to any of (1) to (13), in which
   when a rotation operation on the oblique image is started, update of display content of the oblique image is stopped and display content immediately before the rotation operation is performed is held, and
   when the rotation operation on the oblique image ends, the oblique image is displayed to have display content according to the cutout region moved and set in response to the rotation operation.
(15) A control device of an endoscope system, the control device including:
   a cutout processing unit configured to cut out a part of an entire image obtained by imaging an entire field of view range of a rigid endoscope in accordance with a cutout region set around a position deviated from a center point of the entire image and generate an oblique image viewed in an oblique direction with respect to an optical axis of the rigid endoscope;
   an acquisition unit configured to acquire an instruction command to move the oblique image in a predetermined rotation direction; and
   a region setting unit configured to move the cutout region around the center point of the entire image in a direction according to the instruction command along an inner periphery of a circle in which the cutout region is inscribed.
(16) A control method including causing a control device of an endoscope system
   to cut out a part of an entire image obtained by imaging an entire field of view range of a rigid endoscope in accordance with a cutout region set around a position deviated from a center point of the entire image and generate an oblique image viewed in an oblique direction with respect to an optical axis of the rigid endoscope,
   to acquire an instruction command to move the oblique image in a predetermined rotation direction, and
   to move the cutout region around the center point of the entire image in a direction according to the instruction command along an inner periphery of a circle in which the cutout region is inscribed.

Note that the present embodiments are not limited to the above embodiments and can be variously modified without departing from the gist of the present disclosure. Further, the effects described in the present specification are merely examples and are not limited, and additional effects may be obtained.

### REFERENCE SIGNS LIST

- 11: Endoscope system
- 12: Display device
- 13: Cart
- 14: Endoscope
- 21: Light source device
- 22: CCU
- 23: Control device
- 31: Camera head
- 32: Rigid endoscope
- 41: Camera head main body
- 42: Endoscope coupler
- 43: Operation switch
- 44: Magnetic encoder
- 51: Lens barrel main body
- 52: Light source connection portion
- 53: Coupler connection portion
- 54: Rotation operation portion
- 55: Magnet
- 71: Operation signal acquisition unit
- 72: Voice recognition unit
- 73: Image processing unit
- 81: Region setting unit
- 82: Cutout processing unit
- 83: Marker generation unit
- 84: Display screen generation unit

## Claims

1. An endoscope system comprising:
a cutout processing unit configured to cut out a part of an entire image obtained by imaging an entire field of view range of a rigid endoscope in accordance with a cutout region set around a position deviated from a center point of the entire image and generate an oblique image viewed in an oblique direction with respect to an optical axis of the rigid endoscope;
an acquisition unit configured to acquire an instruction command to move the oblique image in a predetermined rotation direction; and
a region setting unit configured to move the cutout region around the center point of the entire image in a direction according to the instruction command along an inner periphery of a circle in which the cutout region is inscribed.

2. The endoscope system according to claim 1, further comprising:
a display screen generation unit configured to generate a display screen displaying at least one of the entire image or the oblique image; and
a marker generation unit configured to generate a region marker indicating the cutout region in the entire image and superimpose and display the region marker on the entire image displayed on the display screen.

3. The endoscope system according to claim 1, further comprising
an endoscope configured to image an operative field at a high angle of view through the rigid endoscope and supply the entire image to the cutout processing unit.

4. The endoscope system according to claim 3, wherein
the endoscope is configured such that the rigid endoscope is connectable to a camera head including an imaging element that images the operative field.

5. The endoscope system according to claim 4, wherein
the rigid endoscope includes a rotation operation portion for inputting a rotation operation to move the oblique image in a vicinity of a connection portion used for connection to the camera head, and
the acquisition unit acquires the instruction command in response to the rotation operation on the rotation operation portion.

6. The endoscope system according to claim 4, wherein
the camera head includes a coupler used for connection to the rigid endoscope,
the rigid endoscope includes a connection portion to be connected to the coupler,
a magnet is incorporated in one of the connection portion and the coupler, and a magnetic encoder is incorporated in the other of the connection portion and the coupler, and
the acquisition unit acquires the instruction command in response to a rotation operation of rotating the rigid endoscope with respect to the camera head.

7. The endoscope system according to claim 1, wherein
the acquisition unit is a voice recognition unit that acquires the instruction command by recognizing content of an utterance from a voice.

8. The endoscope system according to claim 1, wherein
the region setting unit switches and sets the cutout region around the position deviated from the center point of the entire image and a cutout region around a position at which a center point of the cutout region matches the center point of the entire image, and
the cutout processing unit generates the oblique image on a basis of the cutout region set around the position deviated from the center point of the entire image and generates a forward image on a basis of the cutout region set around the position at which the center point of the cutout region matches the center point of the entire image.

9. The endoscope system according to claim 1, wherein
an angle of view of the entire image is approximately 140° or approximately 120°, and a resolution of the entire image is equal to or more than 4096 × 2304 dots.

10. The endoscope system according to claim 1, wherein
distortion correction is performed on the oblique image in accordance with a position of the cutout region with respect to the entire image.

11. The endoscope system according to claim 1, wherein
the center point of the entire image is a center of an effective area of an image captured through the rigid endoscope.

12. The endoscope system according to claim 1, wherein
the cutout region is set so that a direction of the oblique image rotates as the cutout region moves, and
display content of the oblique image is displayed so as to be vertically inverted depending on a position of the cutout region.

13. The endoscope system according to claim 12, wherein
the display content of the oblique image is vertically inverted depending on whether the position of the cutout region is above or below a horizontal line passing through the center point of the entire image.

14. The endoscope system according to claim 1, wherein
when a rotation operation on the oblique image is started, update of display content of the oblique image is stopped and display content immediately before the rotation operation is performed is held, and
when the rotation operation on the oblique image ends, the oblique image is displayed to have display content according to the cutout region moved and set in response to the rotation operation.

15. A control device of an endoscope system, the control device comprising:
a cutout processing unit configured to cut out a part of an entire image obtained by imaging an entire field of view range of a rigid endoscope in accordance with a cutout region set around a position deviated from a center point of the entire image and generate an oblique image viewed in an oblique direction with respect to an optical axis of the rigid endoscope;
an acquisition unit configured to acquire an instruction command to move the oblique image in a predetermined rotation direction; and
a region setting unit configured to move the cutout region around the center point of the entire image in a direction according to the instruction command along an inner periphery of a circle in which the cutout region is inscribed.

16. A control method comprising causing a control device of an endoscope system
to cut out a part of an entire image obtained by imaging an entire field of view range of a rigid endoscope in accordance with a cutout region set around a position deviated from a center point of the entire image and generate an oblique image viewed in an oblique direction with respect to an optical axis of the rigid endoscope,
to acquire an instruction command to move the oblique image in a predetermined rotation direction, and
to move the cutout region around the center point of the entire image in a direction according to the instruction command along an inner periphery of a circle in which the cutout region is inscribed.
